# EUROPEAN PATENT APPLICATION

(11) **EP 4 364 717 A1**
(43) Date of publication of application: **08.05.2024**
(21) Application number: 23206310.7
(22) Date of filing: 27.10.2023
(51) Int. Cl.: A61J 7/00

(54) **HOME DRUG DISTRIBUTOR AND METHOD FOR CONTROLLING THE ADMINISTRATION OF DRUGS**

(30) Priority: 02.11.2022 IT 202200022485; 28.09.2023 IT 202300020037
(71) Applicant: Sherpa S.r.l., 25020 Dello, Brescia (IT)
(72) Inventor: QUADRINI, Leonardo, I-25020 Dello, BRESCIA (IT)
(74) Representative: Chimini, Francesco

(57) **Abstract**

A home drug distributor (1; 1000), comprises a plurality of cartridges (10; 1010) adapted to contain at least one drug (2) in the form of a pill, lozenge, capsule or tablet, wherein at least one drug outlet opening (22) is obtained in each cartridge (10; 1010). Each cartridge is removably insertable into a cartridge-holder drum (12; 1012). An electronic processing unit (1016) controls electromechanical drug dispensing means (32; 1032) in accordance with a predetermined therapeutic plan.

## Description

The present invention relates to a home distributor of drugs, in particular pills, lozenges or tablets, according to a predetermined administration program. The invention further relates to a method for controlling the correct administration of drugs, parapharmaceuticals, supplements, in the form of pills or lozenges, to a patient by means of the home distributor.

Dispensers of solid drugs, such as pills, capsules or tablets, are known in from the prior art.

These distributors can be used by patients who take one or more drugs at predetermined time intervals: the distributor thus works as both a reminder to not forget to take the prescribed drugs, and as a distributor for taking the prescribed amount of the related drug.

The distributors are configured so that the drugs are authorized based on a predetermined administration program. In case of non-autonomous patients and of patients in home or residential care, the drug distributors can also prevent an inappropriate or non-optimal use of drugs (drug overdose).

In some embodiments, the distributors comprise a base on which a drug container is placed, for example in the form of a rotating carousel. The container is provided with a plurality of compartments in which the drugs have been inserted. The container is rotatable with respect to the base so as to align a compartment with drug dispensing means, according to the predetermined therapeutic plan. In some embodiments, the distributor is connectable to remote devices and can be provided with a screen to allow third parties (family members, pharmacy, hospital, health service, etc.) to remotely control the intake of the drugs.

Examples of home drug distributors are described in US 11433000B2, US200302424943A1, WO2014018499A1, KR 10-1953750, CN104905962A, US8195330B2, WO2021069655A1.

However, in all the known embodiments, the patient or a family member must handle the drugs, or in any case the containers in which the drugs have previously been loaded by another subject, for example a health service provider, so as to load the individual compartments with the drugs, according to the predetermined therapeutic plan.

For example, US 11433000B2 describes a home drug distributor of the aforementioned type. The distributor has a container-holder tray adapted to house a plurality of drug containers. The container-holder tray with the containers is closed above by a lid. The lid is provided with a movable partition which allows access from above to only one container at a time according to the therapeutic plan. In an embodiment described in this publication, the drugs can also be loaded by providing the patient with the containers already filled with drugs. However, since the distributor is already provided with a lid which closes all the containers, the patient must remove the lid of the single pre-loaded container before introducing it into the container-holder tray.

It is apparent that any handling of the drugs or containers by the patient can involve risks of altering the drug administration plan.

It is the object of the present invention to suggest a home drug distributor which combines constructional simplicity, reliability, and ease of use.

It is another object of the present invention to suggest a method for controlling the correct administration of drugs, parapharmaceuticals, supplements in the form of pills or lozenges to a patient, in accordance with the predetermined and personalized therapeutic plan for the individual patient.

Such objects are achieved with a home drug distributor according to claim 1 and a method according to claim 13. The dependent claims describe preferred or advantageous embodiments of the invention.

The features and advantages of the home drug distributor and method for controlling the administration of drugs according to the invention will be in any case apparent from the following description of preferred embodiments thereof, given by way of non-limiting indication, with reference to the accompanying drawings, in which:
- figure 1 is an exploded perspective view of the home drug distributor according to the invention;
- figure 2 is a perspective view of the assembled drug distributor, with an upper lid in phantom;
- figures 3, 3a, 3b and 3c are an exploded perspective view, a front view, a side view, and a bottom perspective view of a drug-holder cartridge of the drug distributor;
- figure 4 is a top view of the cartridge seats of the drug distributor;
- figures 5 and 5a are two perspective views of the drug distributor with the main body in phantom so as to display a drug dispensing mechanism in a first embodiment;
- figures 6 and 6a are two views similar to the preceding ones, with the dispensing mechanism in a second embodiment;
- figures 7 and 7a are two views similar to the preceding ones, with the dispensing mechanism in a third embodiment;
- figures 8 and 8a are two diagrammatic views of the mechanism only in the third embodiment and in two different positions;
- figure 9 is an elevation view of a home drug distributor, in accordance with an embodiment variant;
- figure 10 is a top plan view of the distributor in figure 9;
- figure 11 is a separate elevation view of the distributor in figure 9;
- figure 12 is a front view of the distributor in figure 9;
- figures 13 and 13a are an elevation view and a plan view of the drug-holder drum of the distributor in figure 9; and
- figure 14 is a flow diagram of the drug administration control method according to the invention.

In said drawings, a home drug distributor 2 according to the invention is indicated by 1; 1000 as a whole. The drugs dispensed can be in particular pills, lozenges, capsules, or tablets.

Below in the present disclosure, only the term "drug" is used for convenience, but the distributor can also be used for the administration of parapharmaceuticals or supplements in the form of pills, lozenges, capsules, or tablets.

Moreover, below in the present disclosure, some elements common to the various embodiments described, or elements equivalent to each other, will be indicated by the same reference numerals.

In a general embodiment, the home drug distributor 1; 1000 comprises a plurality of cartridges 10; 1010. Each cartridge forms a plurality of compartments 24; 1024. Each compartment 24; 1024 is adapted to contain at least one drug 2.

Each cartridge 10; 1010 is provided with at least one lid 20 arranged to close the plurality of compartments 24; 1024. The, or each, lid 20 is removable to allow inserting the drugs into the respective compartments 24; 1024.

A least one drug outlet opening 22 is obtained in each cartridge 10; 1010, the outlet opening being adapted to allow at least one drug to exit from at least one compartment when the compartments are closed by the lid 20.

In other words, the drug outlet opening 22 is distinct from the drug insertion opening in the compartments 24; 1024 in the absence of a lid 20.

The distributor 1; 1000 further comprises a cartridge-holder drum 12; 1012 defining a plurality of cartridge housings 26; 1026.

Each cartridge housing 26; 1026 is adapted to removably receive a respective cartridge 10; 1010 provided with a lid.

In particular, the cartridge housings 26; 1026 are open at least on one side to allow an easy insertion and removal of the cartridges 26; 1026.

The cartridge-holder drum 12; 1012 can thus lack a closure lid of the cartridge housings 26; 1026, as the individual cartridges 12; 1012 are already provided with a lid 20 thereof.

Moreover, as mentioned above and as will be better described in the following, during use the cartridges 12; 1012 present in the respective cartridge housings 26; 1026 do not require the removal of any cover for the withdrawal of the drugs, as the drugs are dispensed in an automated manner through the specific drug outlet opening 22.

At least one drug passage duct 28 is obtained in the cartridge-holder drum 12; 1012.

The cartridge-holder drum 12; 1012 is supported by a base 14; 1014. At least one drug dispensing duct 30; 1030 is obtained in the base.

An electronic processing unit 1016 is housed in the base 14; 1014.

The home drug distributor 1; 1000 is provided with electromechanical drug dispensing means controlled by the electronic processing unit to put at least one drug outlet opening 22 of the at least one cartridge 10; 1010 in communication with the or a respective drug passage duct 28 and with the drug dispensing duct 30 in accordance with a predetermined therapeutic plan, e.g., stored in the electronic processing unit.

In some embodiments, the electromechanical drug dispensing means are then configured to:
- check the opening/closing of the drug outlet openings 22, and/or
- move the compartments 26; 1026 so as to replace an empty compartment with a compartment loaded with the drug to be dispensed; and/or
- move the cartridges 10; 1010; and/or
- move the cartridge-holder drum 12; 1012 with respect to the base 14; 1014.

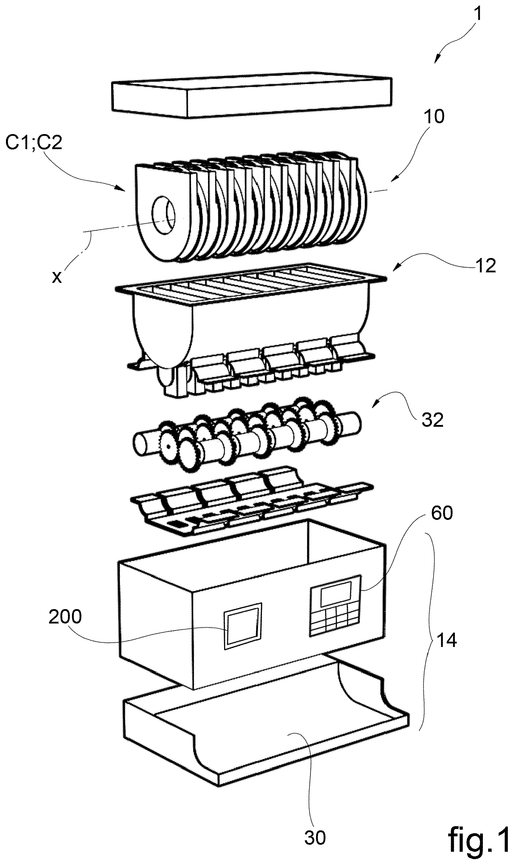

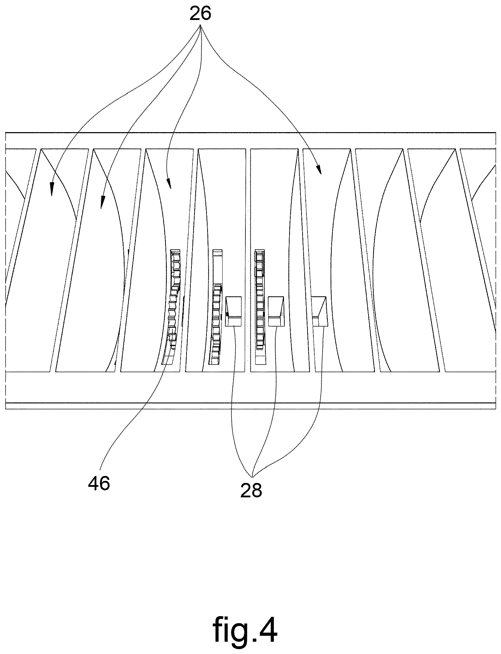

A drug distributor 1 according to the invention, in an embodiment, will now be described with reference to figures 1-8.

The home drug distributor 1 comprises a plurality of cartridges 10, a cartridge-holder drum 12 and a base 14 supporting the cartridge-holder drum 12.

Each cartridge 10 comprises a cartridge body 16 and at least one drug reservoir 18.

The cartridge body 16 is provided with at least one removable lid 20, for example made of transparent plastic material. Each removable lid 20 is applicable to close one or more of the drug reservoirs 18.

At least one drug outlet opening 22 is obtained in a side wall 16' of the cartridge body 16.

As mentioned above, the removable lid 20, when arranged to close the drug reservoir 18, does not engage the drug outlet opening 22.

The drug reservoir 18 has an annular shape and is rotatably housed in the cartridge body 16.

A plurality of compartments 24 is obtained in the drug reservoir 18. Each compartment 24 is adapted to contain at least one drug 2 in the form of a pill, lozenge, capsule or tablet.

In the embodiment shown, each drug reservoir 18 comprises a base 182 and a plurality of radial walls 184 dividing the drug reservoir 18 into a plurality of circular crown sectors forming the compartments 24 of the drug reservoir 18.

In a shown embodiment, one of the circular sectors 24, indicated by 24', is a blind sector, i.e., closed at least radially outwards. In a starting configuration of the cartridges 10 in the drug distributor 1, and further suitable during the transposition of the cartridges loaded with the drugs, such a blind sector 24' is located at the drug outlet opening 22 so as to prevent the unwanted escape of a drug 2 from the cartridge 10.

The drug reservoir 18 is rotatable so as to selectively align the compartments 24 with the drug outlet opening 22.

The cartridge-holder drum 12 defines a plurality of cartridge housings 26. Each cartridge housing 26 is adapted to removably receive a respective cartridge 10 provided with one or more removable lids 20.

A drug passage duct communicating with all the drug outlet openings is obtained in the cartridge-holder drum 12.

In an alternative embodiment shown in the drawings, in the cartridge-holder drum 12, in particular in the bottom wall of the cartridge housings 26, a plurality of drug passage ducts 28 is obtained, each communicating with a respective drug outlet opening 22.

In an embodiment, the one or more drug passage ducts 28 open into a drug dispensing mouth, or duct 30.

A housed electronic processing unit - not shown - and a reservoir actuation assembly 32 are housed in the base 14.

In an embodiment, a control software of the home distributor 1 is stored in the electronic processing unit. In particular, such control software presides over the control of the reservoir actuation assembly 32 in accordance with a predetermined therapeutic plan, i.e., a predetermined pharmacological therapy, for example also stored in the electronic processing unit.

The reservoir actuation assembly 32 is operatively connected to each drug reservoir 18 to cause the selective rotation of one or more reservoirs or the simultaneous rotation of all the drug reservoirs 18.

To this end, the reservoir actuation assembly 32 comprises at least one motor apparatus 34, 35 and a transmission assembly 36 operatively connecting the at least one motor apparatus 34, 35 to at least one drug reservoir 18.

Each motor apparatus 34, 35 is controlled by the electronic processing unit to control the rotation of at least one drug reservoir 18 so as to position a compartment 24 thereof at the drug outlet opening 22 based on the predetermined therapeutic plan.

In an embodiment, the cartridge housings 26 of the cartridge-holder drum are arranged side by side so that the cartridges 10, when inserted into the respective cartridge housings 26, are coaxial with respect to a common rotation axis X.

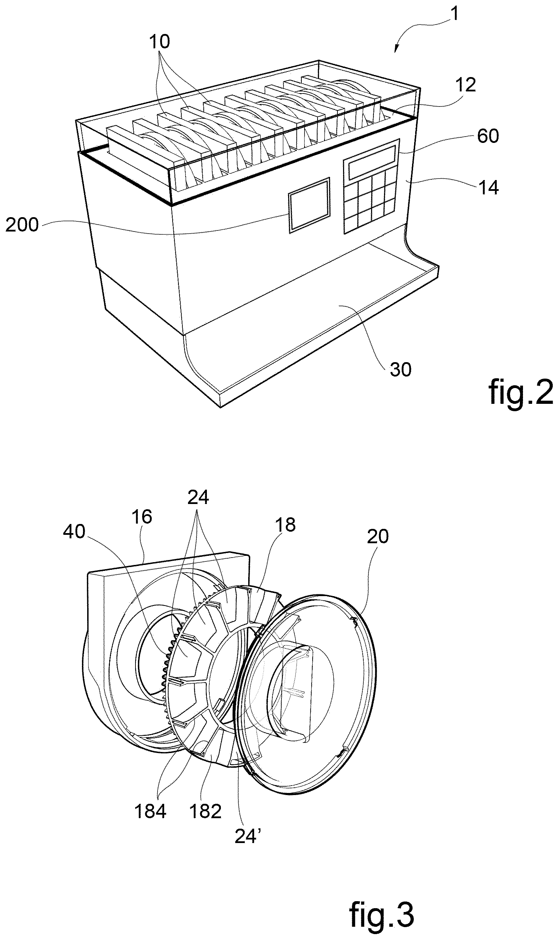

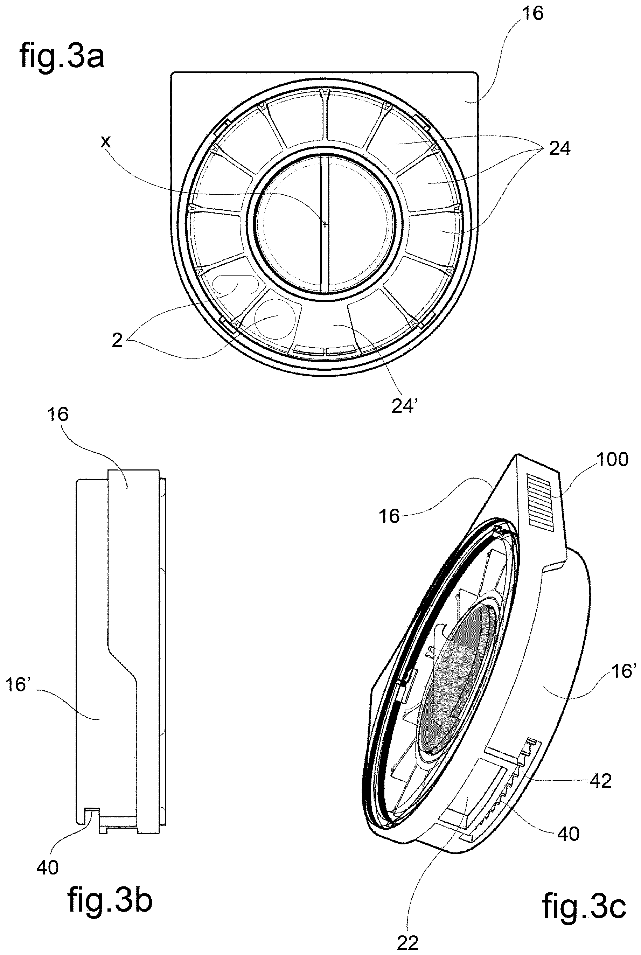

In an embodiment, each drug reservoir 18 is provided, on a circular edge thereof, with a toothed reservoir crown 40. As shown in particular in figure 3c, a crown opening 42 is obtained in the side wall 16' of the cartridge body 16. The transmission assembly 36 comprises at least one transmission shaft 44 rotationally actuatable by a respective motor apparatus 34 and provided with at least one toothed shaft crown 46 adapted to engage the teeth of the toothed reservoir crown 40 through the crown opening 42.

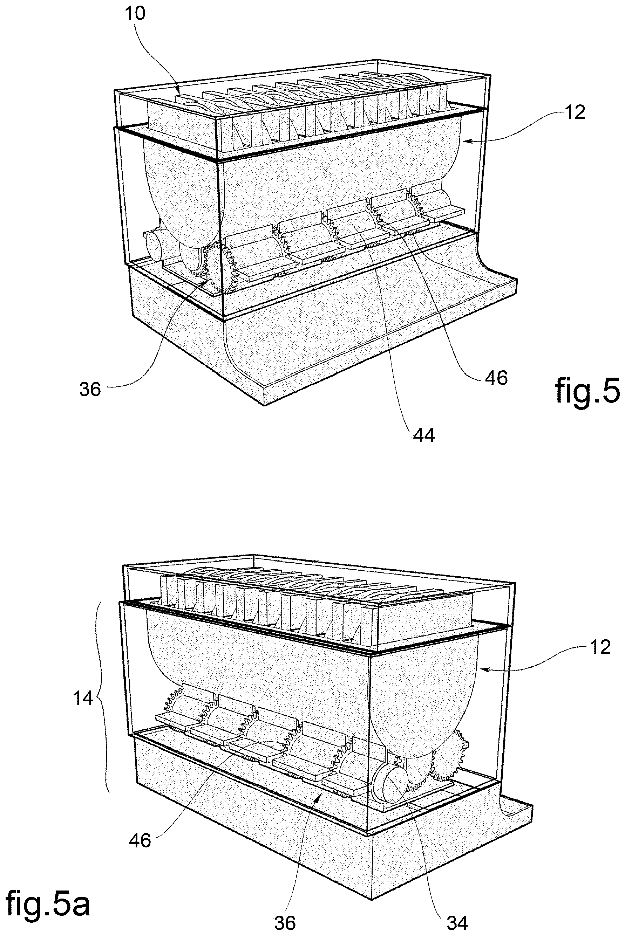

In an embodiment shown in figures 5 and 5a, the reservoir actuation assembly 32 comprises a plurality of transmission shafts 44 rotatably actuatable by respective motor apparatuses 34. Each transmission shaft 44 is provided with a toothed shaft crown 46 adapted to engage the teeth of a respective toothed reservoir crown 40.

Therefore, in this embodiment, a respective motor 34 is associated with each cartridge 10. This technical solution has the advantage that the cartridges can be rotated independently. This is reflected in a short drug dispensing time and in a programming simplicity of the distributor.

In particular, the dispensing time is independent of the starting situation and the number of drugs to be dispensed which are stored in different cartridges, as the selected cartridges can rotate simultaneously and must always rotate only by an angle equal to the width of a sector of the reservoir to emit the next drug.

The cartridges being independent and the relative position irrelevant, the cartridges can also be loaded or removed in any position, without having to move the other cartridges.

The programming of the machine is simple since, knowing the position of the cartridge relating to the drug to be dispensed in the cartridge-holder drum, it is sufficient to rotate it by an angle equal to the width of the sector at the appropriate time.

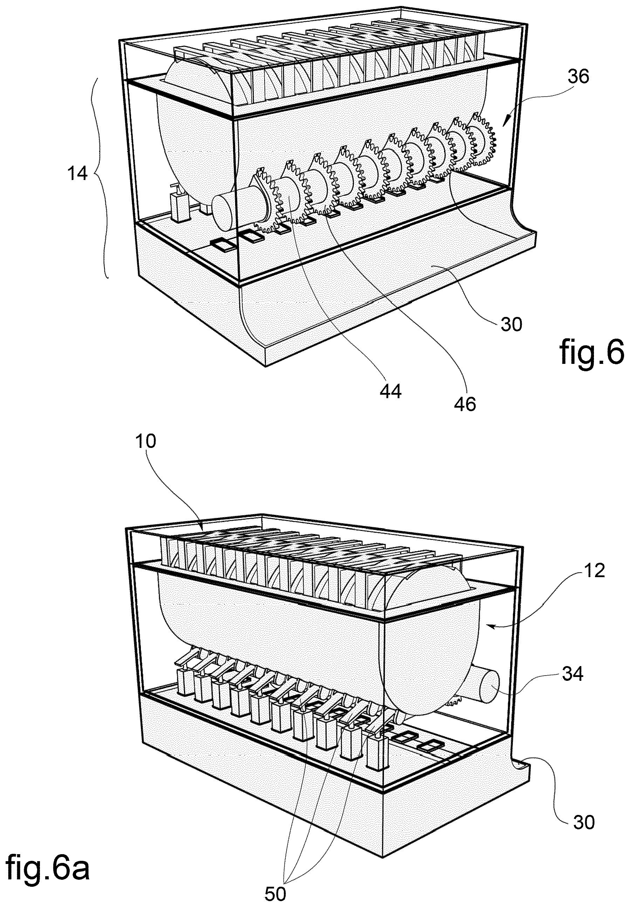

In an embodiment shown in figures 6 and 6a, the actuation assembly is provided with a single transmission shaft 44 provided with a plurality of toothed shaft crowns 46, each engaging a respective toothed reservoir crown 40.

In this case, the actuation assembly can comprise, for each cartridge housing, an electric opening control actuator 50 selectively controllable by the electronic processing unit to open or close the drug outlet opening 22 in accordance with the therapeutic plan stored in the electronic processing unit.

For example, each electric actuator 50 controls the opening/closing of a respective door associated with a respective drug outlet opening 22.

Therefore, all the cartridges are rotated simultaneously and the drug dispensing is controlled through the individual doors.

This technical solution has the advantage of requiring only one transmission shaft actuated by only one motor. However, as compared to the embodiment in figures 5 and 5a, this embodiment involves greater dispensing times and more complex distributor programming.

In particular, this technical solution requires that the cartridges must always be in the same position before a dispensing request.

Therefore, during the loading/removal steps it is necessary to return all the cartridges to the starting position (for example with the blind sector at the drug outlet opening), requiring a wait by the user which in the worst case is equal to the time required to completely rotate the reservoir.

The distributor programming requires storing which sectors have already been emptied for each cartridge. Moreover, the electronic processing unit must be programmed to calculate the minimum rotation which each tank must complete to position the first full sector at the drug outlet opening.

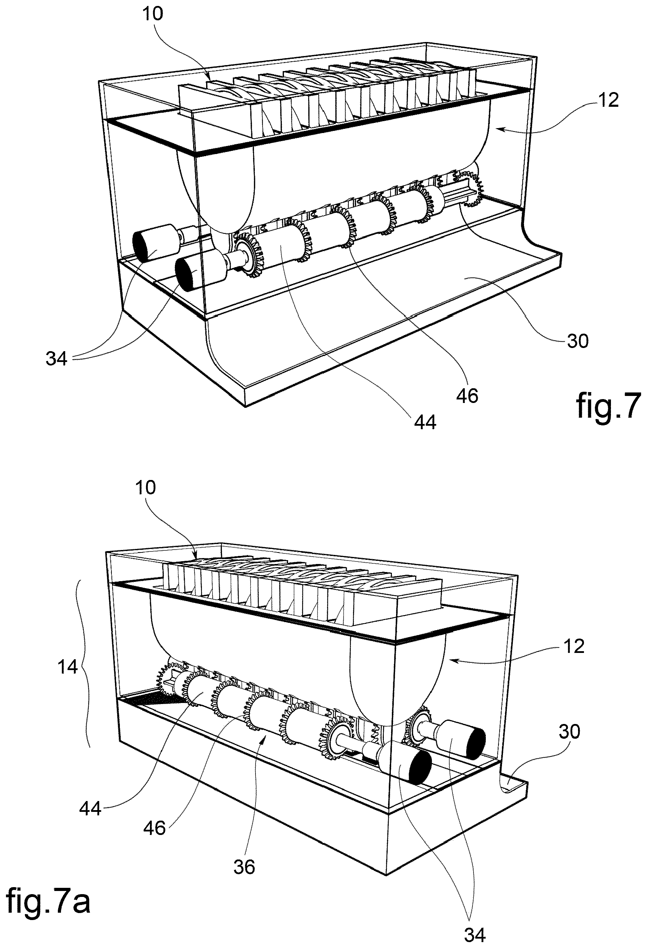

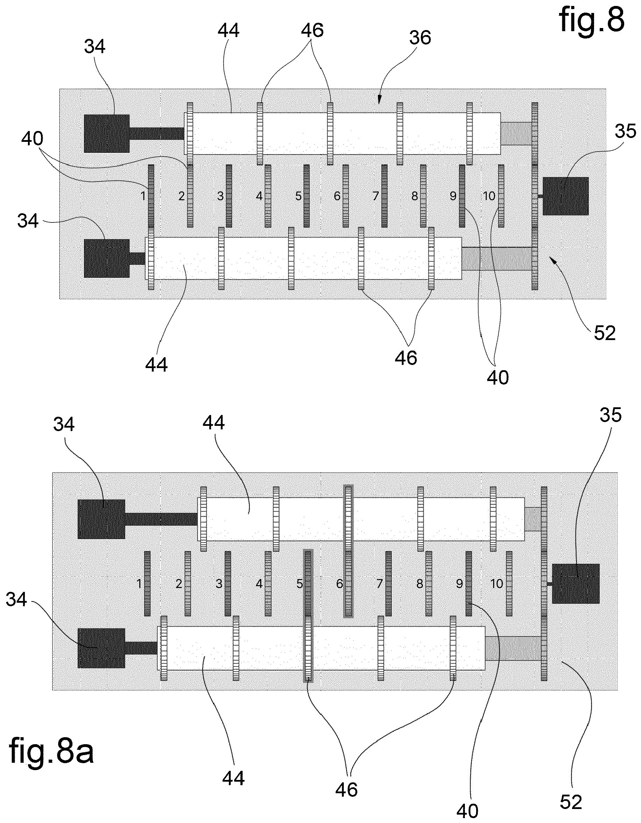

In an embodiment shown in figures 7 and 7a, 8 and 8a, the actuation assembly 32 comprises at least one transmission shaft 44 (two parallel transmission shafts, in the example shown), each provided with a lower number of toothed shaft crowns 46 than the number of toothed reservoir crowns 40. The transmission assembly 36 comprises axial shaft control means 52 adapted to cause an axial translation of each transmission shaft 44 so as to selectively position at least one toothed shaft crown 46 at a toothed reservoir crown 40.

For example, such axial control means 52 comprise a ball screw system, in which the screw is rotationally actuated by a respective screw motor 35 controlled by the electronic processing unit.

In the example in figures 8 and 8a, the distributor is provided with ten cartridges 10, of which only the related toothed wheels 40 are visible, and with two transmission shafts 44, each provided with five toothed shaft crowns 46.

In figure 8, the two transmission shafts 44 are positioned axially so as to engage the first two toothed wheels 40.

In figure 8a, the two transmission shafts 44 have been translated axially so as to engage the fifth and the sixth toothed wheel 40.

In this embodiment, since the cartridges are independent and the relative position is irrelevant, the cartridges can be loaded or removed in any position, without having to move the other cartridges.

The distributor programming is relatively simple since, knowing the position of the cartridge relating to each drug, it is sufficient to rotate the tank by an angle equal to the width of a sector, as in the first embodiment described above. As compared to the first embodiment, the dispensing time can be greater as it can be necessary to axially translate one or more transmission shafts before rotating the toothed wheels.

In an embodiment, the distributor 1 is further provided with a user interface 60 adapted to allow, for example to an authorized subject having access to the medical prescription including the therapeutic plan, programming the control software with input data necessary to customize the activation and control of the reservoir actuation assembly 32 according to the therapeutic plan.

In an embodiment, a refrigerant system - not shown - adapted to maintain the temperature of the drugs contained in the cartridges below a predetermined temperature is housed in the base 14.

In an embodiment, the distributor 1 is further provided with a reader 200, for example a barcode or QR-code reader, adapted to read a cartridge identification code 100. As will be described in the following, such a cartridge identification code 200 is associated with each cartridge 100 to allow the correct positioning of the cartridge 10 in the cartridge-holder drum 12 and thus the dispensing of the drugs in accordance with a predetermined therapeutic plan.

The distributor 1 can also be provided with signaling means adapted to indicate to the user the housing in which the cartridge is to be inserted when the cartridge identification code is read by the reader 200. For example, the signaling means comprise a light indicator positioned at each cartridge housing 26.

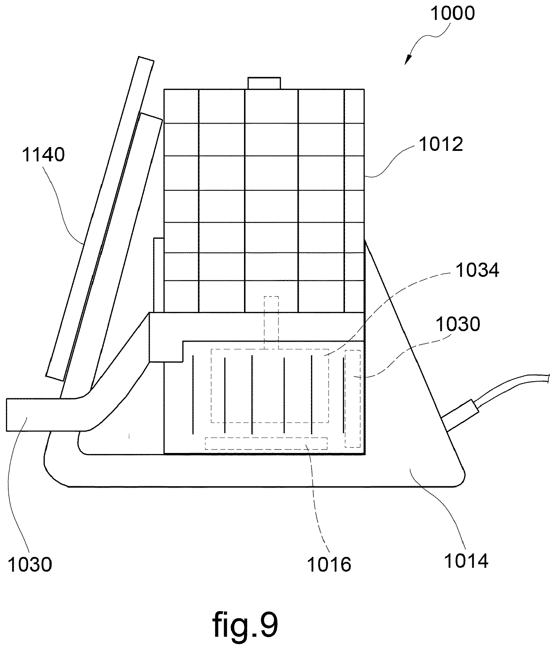

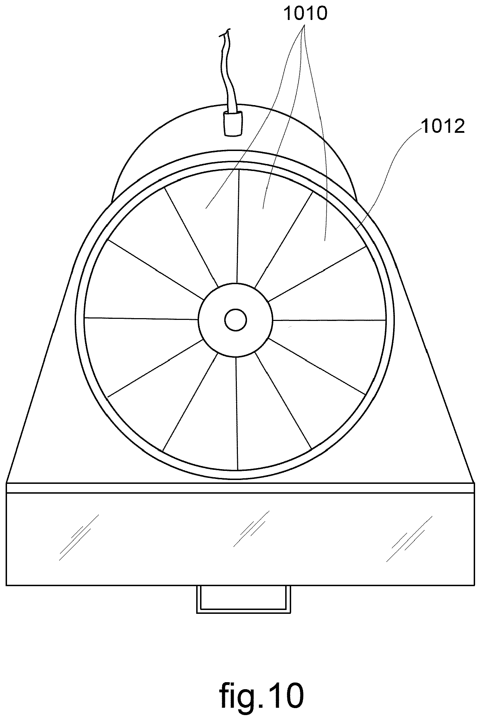

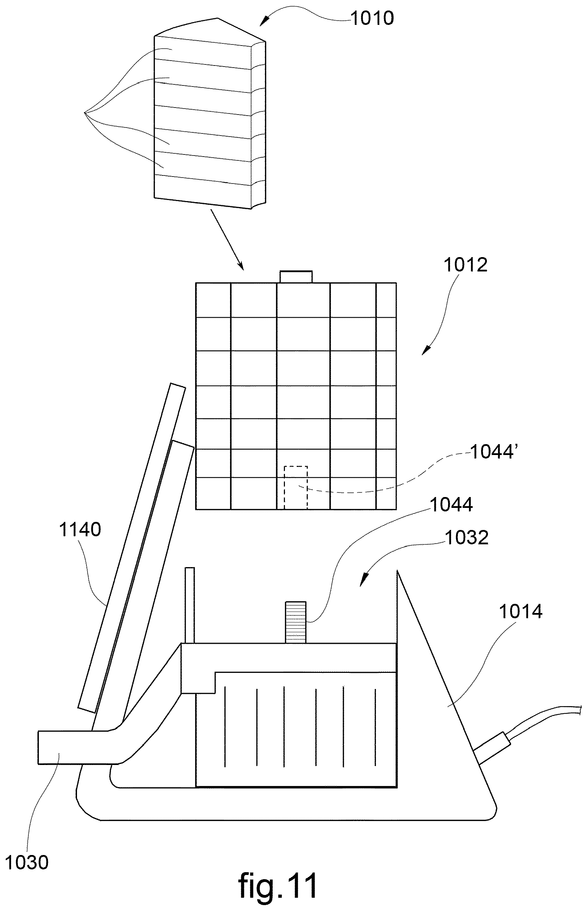

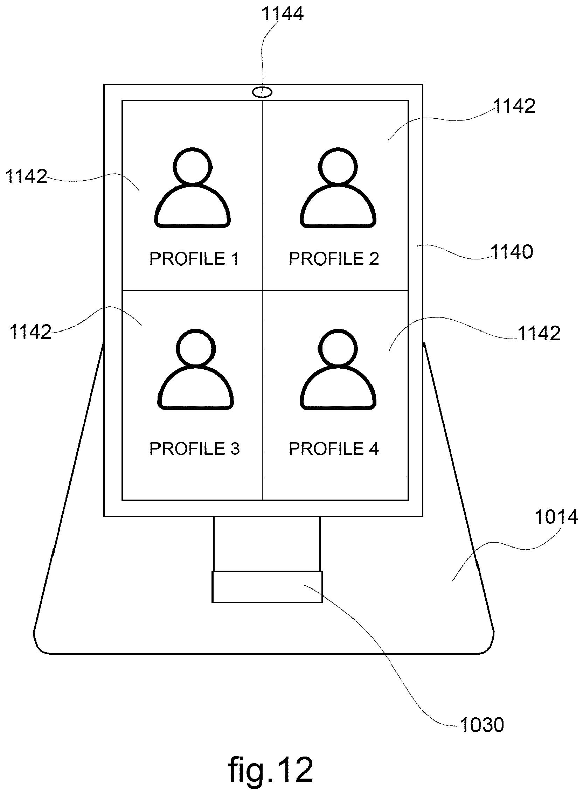

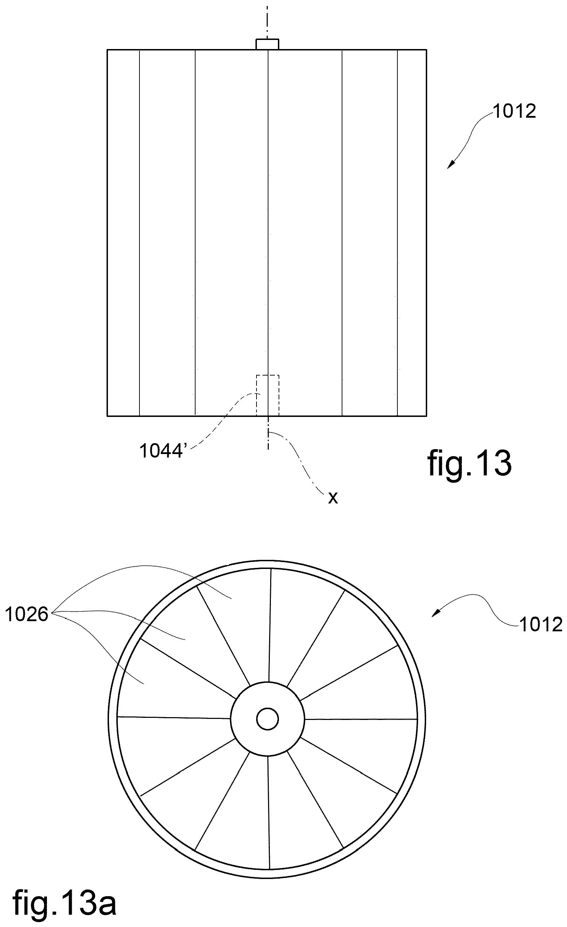

With reference to figures 9-13, a home drug distributor 1000 according to the invention will now be described, in a possible alternative embodiment.

The home drug distributor 1000 comprises a base 1014, a drug-holder drum 1012 adapted to be supported by the base 1014, electromechanical drug dispensing means 1032, and an electronic processing unit 1016 housed in the base 1014.

The base 1014 is adapted to be rested on a support surface.

A motor apparatus 1034 controlled by the electronic processing unit 1016 and provided with a motor shaft 1044 is housed in the base 1014.

In an embodiment, the drug-holder drum 1012 and the base 1014 are provided with releasable mutual coupling means - not shown - for example of a mechanical, electromechanical, or magnetic type.

In other words, the drug-holder drum 1012 is separable from the base 1014 to be transported, as will be described in the following, to a subject responsible for loading the drugs 2 prescribed by the therapeutic plan, for example an authorized pharmacy.

The drug-holder drum 1012 is adapted to be operatively connected to the transmission shaft 1044 when the drug-holder drum 1012 is coupled to the base 1014.

The home distributor 1000 is provided with a plurality of cartridges 1010 for containing drugs 2. Each cartridge 1010 is adapted to be removably inserted into a respective cartridge housing 1026 obtained in the drug-holder drum 1012.

For example, the drug-holder drum 1012 has a cylindrical structure extending about a drum rotation axis X. The cartridge housings 1026 are evenly distributed about the drum rotation axis X. Each cartridge housing 1026 can have the upper side open so as to allow the insertion of a respective cartridge 1010.

For example, the cartridge housings 1026 have a height which is substantially equal to the height of the drug-holder drum 1012 and have a cross-section in the form of a circular sector or circumference wedge.

The cartridges 1010 can have a shape which is substantially complementary to that of the respective cartridge housings 1026, i.e., have a prismatic structure.

In an embodiment, the drug-holder drum 1012 forms an axial cavity 1044' configured for a shape coupling with an end portion of the motor shaft 1044.

Each cartridge 1010 is divided into compartments 1024, each adapted to contain at least one drug 2.

For example, in an embodiment in which the drug-holder drum 1012 is arranged with the drum rotation axis X oriented vertically, each cartridge 1010 is vertically divided into compartments 1024, which are then overlapped, or stacked, together.

The drug-holder drum 1012, when coupled to the base 1014, is rotatable by the motor apparatus so as to position a cartridge 1010 at a drug dispensing duct 1030, for example in the form of a chute, obtained in the base 1014. Therefore, the drug(s) exiting from a compartment 1024 exit from the base 1014 by gravity.

In some embodiments, the distributor 1000 is provided with a drug release system from the compartments 1024 or an opening/closing system of the compartments 1024, such systems being for example of a mechanical or electrical or electromechanical type.

In an embodiment, a refrigerant system 1130 adapted to maintain the temperature of the drugs contained in the drug-holder drum 1012 below a predetermined temperature is housed in the base 1014.

In an embodiment, a screen 1140 controlled by the electronic processing unit 1016 is connected to the base 1014.

In an embodiment, multiple therapeutic plans are stored in the electronic processing unit 1016. The distributor 1000 is provided with a user interface, which can be implemented from the same screen 1140 if made as a touch screen. The user interface can implement selector means operable by a user to select one or more therapeutic plans.

Therefore, it is possible, on the same home distributor 1000, to create multiple user profiles 1142 and associate a therapeutic plan with each of them.

In an embodiment, the electronic processing unit 1016 is provided with data transmission means configured to send to an authorized pharmacy the therapeutic plan stored in the processing unit 1016 and a unique identification code (ID code) which uniquely identifies the patient and/or the home distributor 1000 provided to the patient.

In an embodiment, such a unique identification code is carried on the drug-holder drum 1012. For example, the unique identification code is a barcode or a QR-code.

In an embodiment, the home distributor 1000 is further provided with sensors for verifying the correct drug release/dosage.

In an embodiment, the home distributor 1000 is managed by control software. Such control software can further be configured to make the home distributor 1000 interact with the patient's reference pharmacy and possibly with an intermediary, provider of a home distributor management service.

The control software notifies and dispenses at the time of taking the drug following the therapeutic plan loaded in the user profile.

Through the screen 1140 and a video camera 1144, the user can make a video connection with the Pharmacist, for example for any doubt about taking the drugs.

Moreover, the control software can be programmed to allow the user to book the refill appointment (at the pharmacy, carrying the drug-holder drum or individual cartridges, or at home) during which the pharmacist also monitors the basic vital parameters (blood pressure, blood glucose saturation and cholesterol) which will be entered and integrated in the user profile.

It is apparent to those skilled in the art that some features of one embodiment described can also be provided for in another embodiment described, and vice versa.

The present invention further relates to a method for controlling the administration of drugs, parapharmaceuticals, supplements, in the form of pills or lozenges, to a patient using the drug distributor 1; 1000 described above.

The home drug distributor 1; 1000 is initially delivered to the patient already provided with a first set of cartridges C1 filled with the prescribed drugs and programmed so as to actuate the predetermined therapeutic plan, i.e., the drug therapy.

The suggested drug administration control method further includes providing an authorized pharmacy F with at least a second set of cartridges C2, i.e., replacement cartridges, comprising one or more cartridges equal to the cartridges of the first set of cartridges C1 housed in the cartridge-holder drum 12; 1012.

Periodically, or close to finishing the drugs contained in one or more of the cartridges 10; 1010 of the first set of cartridges C1, the staff of the authorized pharmacy F fills the cartridges of the second set of cartridges C2 corresponding to the finished cartridges of the first set of cartridges C1 with the prescribed drugs in accordance with the predetermined therapeutic plan.

For example, such a filling is carried out with the support of a filling software SW programmed based on the therapeutic plan. The filling software SW is configured to associate each filled cartridge with a respective position in the home distributor. For example, the position of the cartridge refers to the position, for example identified by a number, of the cartridge housing 26; 1026 of the cartridge-holder drum 12; 1012 in which the cartridge must be inserted.

Once each cartridge has been assigned a respective position, the information related to such a position is made accessible by associating each filled cartridge of the second set of cartridges with an identification element containing position information related to the position of the cartridge in the home distributor.

For example, the identification element consists of a label 100 on which a barcode or QR-code is printed and which is affixed to the cartridge.

The finished cartridges of the first set of cartridges C1 can thus be replaced by the corresponding replacement cartridges of the filled second set of cartridges C2.

For example, the patient delivers one or more finished cartridges of the first set of cartridges C1 to the authorized pharmacy F, picks up the corresponding filled replacement cartridges C2, and inserts the replacement cartridges C2 into the home distributor 1.

In an embodiment, the replacement operation occurs through the reading of the position information, which allows inserting the filled cartridges into the respective housings, in accordance with the therapeutic plan stored in the electronic processing unit.

In other words, the filling software is programmed to generate cartridge identification codes containing the position information of each cartridge. Each cartridge identification code is printed on a label 100 to be affixed to a respective cartridge, and the home distributor is provided with a cartridge identification code reader 200.

For example, reading the position information causes the activation of a visual indication of the housing of the cartridge-holder drum in which a respective cartridge must be inserted, for example the emission of a light signal at the correct housing.

In an embodiment, the filling software SW generates a set of virtual cartridges CV (possibly also in combination with the cartridge-holder drum) and simulates the filling of the second set of cartridges C2, in accordance with the patient's therapeutic plan. Optionally, the insertion of the second set of cartridges in the virtual cartridge-holder drum is also simulated, if the patient also delivers the cartridge-holder drum to the authorized pharmacy and the authorized pharmacy F provides for the replacement of the cartridges in the cartridge-holder drum.

By virtue of the matching between the set of second cartridges C2 which must be filled and the set of virtual cartridges CV, the filling operation is simplified and the risk of making a mistake in filling the cartridges is highly reduced.

In an embodiment, the authorized pharmacy F receives the therapeutic plan from the patient himself. In other embodiments, the authorized pharmacy F receives the therapeutic plan from an intermediary subject I in possession of a proxy contract D stipulated with the patient.

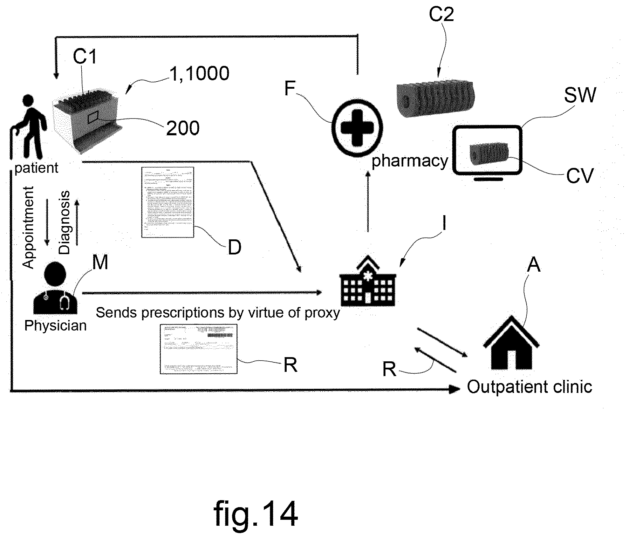

For example, as shown in the flow diagram in figure 14, the intermediary subject I receives the prescription R, containing the therapeutic plan, from the family physician M or the physician of an outpatient clinic A where examinations prescribed by the physician are carried out.

In some embodiments, the intermediary subject I, equipped with the patient's proxy D, manages the drug administration service and selects and controls the pharmacies F which adhere to such a service. For example, the intermediary subject I can be assigned to receive the prescriptions, provide the authorized pharmacies with the therapeutic plan included in or derived from the prescriptions, develop and provide the authorized pharmacies with the filling software, program the home drug distributor based on the therapeutic plan.

Moreover, the intermediary subject I can manage a cloud server to which users of home distributors can connect (for example by means of a home distributor management app or through the distributor itself), authorized pharmacies and possibly other health professionals, to remotely assist users in case of difficulties in using the home distributor, and possibly to modify the therapeutic plan.

For example, the control software can also receive control parameters of the drug dispensing means from the server at the time of administration of a drug.

In some embodiments, the control software which oversees the control of the home distributor allows creating multiple user profiles, differentiating them for example by means of bright colors at the time of prescription administration.

The control software can also allow direct video consultation (by means of monitor and camera) with a physician, the pharmacy and with support.

By virtue of the suggested home distributor and drug administration control method, it is possible to obtain or improve:
- adherence to therapy (compliance) and therefore a greater effectiveness thereof, with a decrease in the risk of prolongation or exacerbation of symptoms;
- decrease in appointments and possible hospitalizations with a decrease in costs for the National Health Service;
- reduction of drug-related waste (blisters and packaging), by virtue of the preparation of cartridges in the galenic laboratory by the authorized pharmacy;
- loyalty of the patient to the pharmacy selected as a reference.

Note that, by virtue of the home distributor and the described cartridge replacement method, the patient is allowed to come into contact with the drugs only when dispensed from the home distributor and not to change the state of the cartridge when replacing a finished cartridge with a replacement cartridge.

In fact, in particular, the patient receives the refilled and closed replacement cartridges from the pharmacy, i.e., provided with a lid. During the replacement of finished cartridges with those of replacement, the patient is not obliged to open the cartridges or carry out other operations on the cartridges, but to insert them into the respective housings, possibly after having the distributor read the code identifying the position thereof.

In addition to the simplicity of use of the home distributor, even for elderly or not completely lucid patients, the risk of incorrect, if not even dangerous, drug intake is thus greatly reduced.

Moreover, the introduction of the suggested home distributor and drug administration control method will allow the creation of a new figure, referred to as the "Family Pharmacist", capable of taking care of a certain number of patients, as occurs for general medical practitioners.

For example, such a new figure may deal in particular with:
- verifying the correct preparation of the cartridge-holder drum, for example on a monthly basis, by means of a software connection with the Pharmacy and with the service management platform;
- by acceding the home distributor management software, monitoring daily drug intake and update the treatment plan;
- updating the dispensing of therapies based on the evolution of the therapeutic plan;
- going physically (if required), or by means of web connection, to the patient's home, for example for the replacement of finished cartridges with replacement cartridges, for activating monthly therapies and for monitoring vital parameters (blood pressure, cholesterol, saturation, heart rate, and blood glucose level) which will be added to the patient's profile.

Those skilled in the art may make changes and adaptations to the embodiments of the home drug distributor and the drug dispensing control method according to the invention or can replace elements with others which are functionally equivalent in order to meet contingent needs without departing from the scope of the appended claims. Each of the features described above as belonging to one possible embodiment can be implemented irrespective of the other embodiments described.

## Claims

1. A home drug distributor (1; 1000), comprising:
- a plurality of cartridges (10; 1010), each cartridge forming a plurality of compartments (24; 1024), each compartment being adapted to contain at least one drug (2) in the form of a pill, lozenge, capsule or tablet, wherein each cartridge is provided with at least one lid (20) arranged to close the plurality of compartments, the lid being removable to allow inserting the drugs into the respective compartments, and wherein at least one drug outlet opening (22) is obtained in each cartridge (10; 1010), the outlet opening being adapted to allow at least one drug to exit from at least one compartment when the compartments are closed by the lid;
- a cartridge-holder drum (12; 1012) defining a plurality of cartridge housings (26; 1026), each adapted to removably receive a respective cartridge (10; 1010) provided with a lid, at least one drug passage duct being obtained in the cartridge-holder drum;
- a base (14; 1014), the cartridge-holder drum (12; 1012) being supported by the base, at least one drug dispensing duct being obtained in the base;
- an electronic processing unit (1016) housed in the base;
- electromechanical drug dispensing means (32; 1032) controlled by the electronic processing unit to put at least one drug outlet opening (22) of the at least one cartridge in communication with the or a respective drug passage duct and with the drug dispensing duct in accordance with a predetermined therapeutic plan, for example stored in the electronic processing unit.

2. A home drug distributor according to claim 1, wherein:
- each cartridge (1010) is formed by a vertical overlap of compartments (1024), a respective drug outlet opening being obtained in a bottom wall of each compartment;
- the cartridge housings (1026) extend radially about a drum axis (X);
- the cartridge-holder drum (1012) is provided with a plurality of drug passage ducts, each aligned with a respective drug outlet opening, and is mounted to the base so as to be rotatable about said drum axis through the electromechanical drug dispensing means to selectively put a drug passage duct in communication with the drug dispensing duct.

3. A home drug distributor according to claim 1, wherein:
- each cartridge (10) comprises:
- a cartridge body (16) provided with at least one lid (20), the at least one drug outlet opening (22) being obtained in a side wall (16') of the cartridge body;
- at least one annular drug reservoir (18) rotatably housed in the cartridge body (16), the plurality of compartments (24) being obtained in the drug reservoir, the lid (20) being applicable to close a respective drug reservoir (18), the drug reservoir (18) being rotatable so as to selectively align the compartments (24) with the drug outlet opening (22);
- a drug passage duct, communicating with all the drug outlet openings, or a plurality of drug outlet ducts (28), each communicating with a respective drug outlet opening (22), being obtained in the cartridge-holder drum;
- the electromechanical dispensing means comprise a reservoir actuation assembly (32) housed in the base and operatively connected to each reservoir (18) to cause the selective rotation of one or more reservoirs or the simultaneous rotation of all the reservoirs, the reservoir actuation assembly (32) comprising at least one motor apparatus (34, 35) and a transmission assembly (36) which operatively connects the at least one motor apparatus (34) to at least one reservoir (18), each motor apparatus being controlled by the electronic processing unit to control the rotation of at least one reservoir so as to position a compartment thereof at the drug outlet opening based on the predetermined therapeutic plan.

4. A distributor according to claim 3, wherein each drug reservoir (18) comprises a reservoir base (182) and a plurality of radial walls (184) dividing the drug reservoir into a plurality of circular crown sectors forming the compartments (24) of the drug reservoir (18).

5. A distributor according to claim 3 or 4, wherein the cartridge housings (26) are placed side by side so that the cartridges, when inserted into the respective cartridge housings, are coaxial with respect to a common rotation axis (X).

6. A distributor according to any one of claims 3-5, wherein each drug reservoir (18) is provided, on a circular edge thereof, with a toothed reservoir crown (40), a crown opening (42) being obtained in the side wall of the cartridge body, wherein the transmission assembly comprises at least one transmission shaft (44) rotationally actuatable by a respective motor apparatus (34) and provided with at least one toothed shaft crown (46) adapted to engage the teeth of the toothed reservoir crown (40) through said crown opening (42).

7. A distributor according to claim 6, wherein the transmission shaft (44) is provided with a plurality of toothed shaft crowns (46), each engaging a respective toothed reservoir crown (40).

8. A distributor according to claim 6, further comprising, for each cartridge housing, an electric opening control actuator (50) selectively controllable by the electronic processing unit to open or close the drug outlet opening (22) in accordance with the predetermined therapeutic plan.

9. A distributor according to claim 6, comprising a plurality of transmission shafts (44) rotationally actuatable by respective motor apparatuses (34), each transmission shaft being provided with a toothed shaft crown (46) adapted to engage the teeth of a respective toothed reservoir crown (40).

10. A distributor according to claim 6, comprising at least one transmission shaft (44) provided with a lower number of toothed shaft crowns (46) than the number of toothed reservoir crowns, the transmission assembly comprising axial shaft control means (52) adapted to cause an axial translation of each transmission shaft (44) so as to selectively position at least one toothed shaft crown (46) at a toothed reservoir crown (40).

11. A distributor according to claim 10, wherein said axial shaft control means (52) comprise a ball screw system, wherein the screw is rotationally actuated by a respective screw motor controlled by the electronic processing unit.

12. A distributor according to any one of the preceding claims, comprising a reader (200) for reading a cartridge identification code (100) associated with each cartridge, and signaling means adapted to activate a light indicator at a cartridge housing (46) when the cartridge identification code is read by the reader.

13. A method for controlling the correct administration of drugs, parapharmaceuticals, supplements in the form of pills, lozenges, capsules or tablets, to a patient, comprising the steps of:
- providing the patient with a home drug distributor (1; 1000) according to any one of the preceding claims, provided with a first cartridge set (C1) inserted into the cartridge-holder drum (12; 1012);
- programming the electronic processing unit based on the patient's therapeutic plan;
- providing an authorized pharmacy (F) with a second cartridge set (C2) comprising at least one replacement cartridge, equal to the cartridges of the first cartridge set (C1);
- periodically, or when the drugs contained in one or more of the cartridges of the first cartridge set (C1) are almost finished, filling, by the authorized pharmacy, one or more replacement cartridges of the second cartridge set (C2) with the drugs prescribed by the therapeutic plan, wherein the filling step is performed with the aid of a filling software (SW) programmed based on the therapeutic plan, the filling software being configured to associate each filled replacement cartridge with a respective position in the cartridge-holder drum;
- associating each filled cartridge of the second cartridge set with an identification element (100) containing position information related to the position of the cartridge in the cartridge-holder drum;
- replacing the empty cartridges of the first cartridge set (C1) with the filled replacement cartridges (C2), wherein the replacement step comprises reading the position information.

14. A method according to claim 13, wherein the filling software generates cartridge identification codes containing the position information of each cartridge, each cartridge identification code being printed on a label to be affixed to a respective cartridge, and wherein the home distributor is provided with a reader (200) for reading said cartridge identification code.

15. A method according to claim 13 or 14, wherein the reading of the position information causes the activation of a visual indication of the housing of the cartridge-holder drum in which a respective cartridge must be inserted.

16. A method according to any one of the preceding claims 13-15, wherein the authorized pharmacy, or an assigned family pharmacist, has access to a patient's therapeutic plan by means of a proxy contract (D) stipulated between the patient and an intermediary subject, the authorized pharmacy receiving the therapeutic plan from said intermediary subject.
